# EUROPEAN PATENT SPECIFICATION

(11) **EP 3 930 593 B1**
(45) Date of publication and mention of the grant of the patent: **04.06.2025**
(21) Application number: 20766953.2
(22) Date of filing: 28.02.2020
(51) Int. Cl.: A61B 17/135, A61B 17/12, A61M 39/02, A61M 39/06, A61B 17/00, A61B 17/132, A61M 39/10

(54) **HEMOSTASIS DEVICES WITH FOLDED BALLOON ASSEMBLIES**
HÄMOSTATISCHE VORRICHTUNGEN MIT GEFALTETEN BALLONANORDNUNGEN
DISPOSITIFS D'HÉMOSTASE À ENSEMBLES DE BALLONNETS PLIÉS

(30) Priority: 01.03.2019 US 201962812436 P
(43) Date of publication of application: 05.01.2022
(73) Proprietor: TERUMO Kabushiki Kaisha, Tokyo 151-0072 (JP)
(72) Inventor: O'BRIEN, Victoria, Moore, Somerset, NJ 08873 (US); HOFFMAN, Brian, Somerset, NJ 08873 (US); HAZAMA, Kenichi, Somerset, NJ 08873 (US); VARAMO, Nicholas, Somerset, NJ 08873 (US)
(74) Representative: Casalonga
(86) International application number: PCT/US2020/020497
(87) International publication number: WO 2020/180730

(56) References cited:
- WO-A1-2018/008600
- US-A1- 2018 042 615
- US-A1- 2018 042 615
- US-A1- 2018 263 634
- US-A1- 2018 310 944
- US-A1- 2019 015 110
- US-B1- 9 393 027

## Description

### CROSS-REFERENCE TO RELATED APPLICATION(S)

This application claims priority to U.S. Provisional Patent Application No. 62/812,436, filed March 1, 2019.

### FIELD OF THE INVENTION

The present invention relates to hemostasis devices (e.g., bands) that are adapted to act as compression devices to promote hemostasis at a surgical access site, and more particularly to hemostasis bands having folded balloon assemblies. In particular, the present invention relates to a balloon assembly for a hemostasis device according to the preamble of claim 1, such as it is e.g. known from US2018042615 A1.

### BACKGROUND

After a surgical procedure involving arterial or venous access, it may be desirable or necessary to apply pressure to the vascular access site to promote hemostasis. Some existing hemostasis devices use one or more inflatable balloons to apply pressure to the access site. In some instances, these balloons have experienced failures. Some existing hemostasis devices may also be time-consuming and expensive to construct. Accordingly, there is a need for hemostasis devices that address these and other drawbacks of the prior art.

### SUMMARY OF THE INVENTION

These problems are solved by a balloon for a hemostasis device according to independent claim 1. The dependent claims relate to advantageous embodiments.

### BRIEF DESCRIPTION OF THE DRAWINGS

The present invention will hereinafter be described in conjunction with the appended drawing figures, wherein like numerals denote like elements.
FIG. 1 is a partial perspective view of a hemostasis device including a balloon assembly according to the prior art, in an uninflated state;
FIG. 2 is a top view of a balloon assembly according to the prior art, in an uninflated state;
FIG. 3 is a perspective side view of a hemostasis device including a balloon assembly according to the prior art, in an inflated state, attached around an arm of a patient;
FIG. 4 is a top perspective view of a hemostasis device according to the present invention including a folded balloon assembly according to one embodiment of the present invention, with the folded balloon assembly shown in an uninflated state;
FIG. 5 is a schematic top view of the balloon assembly of the embodiment of FIG. 4, shown unfolded while in an uninflated state;
FIG. 6 is a top perspective view of the balloon assembly of the embodiment of FIG. 4, shown folded while in its uninflated state;
FIG. 7 is a side view thereof;
FIG. 8 is a top perspective view of a hemostasis device including the balloon assembly of the embodiment of FIG. 4, shown folded while in an inflated state;
FIG. 9 is a perspective side view thereof;
FIG. 10 is a perspective side view of the hemostasis device of FIG. 4, with the balloon assembly in an inflated state, attached around an arm of a patient;
FIG. 11 is a top view of a folded balloon assembly according to another embodiment of the present invention, in an uninflated state;
FIG. 12 is a schematic top view thereof, shown unfolded while in an uninflated state;
FIG. 13 is a perspective side view of the balloon assembly of FIG. 11, shown in an inflated state, used as part of a hemostasis device which is attached around an arm of a patient;
FIG. 14 is a top view of a folded balloon assembly according to another embodiment of the present invention, in an uninflated state;
FIG. 15 is a schematic top view thereof, shown unfolded while in an uninflated state;
FIG. 16 is a perspective side view of the balloon assembly of FIG. 14, shown in an inflated state, used as part of a hemostasis device which is attached around an arm of a patient;
FIG. 17 is a top view of a folded balloon assembly according to another embodiment of the present invention, in an uninflated state;
FIG. 18 is a schematic top view of a folded balloon assembly according to yet another embodiment of the present invention, shown unfolded while in an uninflated state;
FIG. 19 is a schematic top view of a folded balloon assembly according to still another embodiment of the present invention, shown unfolded while in an uninflated state;
FIG. 20 is a sectional view of a balloon assembly according to the prior art;
FIG. 21 is a sectional view of a balloon assembly according to the present invention; and
FIGS. 22 and 23 are schematic views of a balloon assembly according to the present invention.

### DETAILED DESCRIPTION OF THE EMBODIMENT(S)

The ensuing detailed description provides exemplary embodiment(s) only, and is not intended to limit the scope, applicability, or configuration thereof. Rather, the ensuing detailed description of the exemplary embodiment(s) will provide those skilled in the art with an enabling description for implementing these embodiment(s). It should be understood that various changes may be made in the function and arrangement of elements of the embodiment(s) without departing from the scope of the invention, as set forth in the appended claims.

Directional terms (e.g., upper, lower, left, right, etc.) may be used herein. These directional terms are merely intended to assist in disclosing the embodiment(s) and claiming the invention and are not intended to limit the claimed invention in any way. In addition, reference numerals that are introduced in the specification in association with a drawing figure may be repeated in one or more subsequent figure(s) without additional description in the specification, in order to provide context for other features.

Peripheral vascular interventions are commonly used to attempt to clear occlusions from, or surgically introduce stents into, vascular pathways. For example, antegrade crossing via the radial artery in a patient's wrist is common, and various retrograde approaches upwardly from below a patient's knee are also established procedures. After such a procedure, the vascular (*i.e.*, either arterial or venous) access site is typically closed through application of pressure to encourage hemostasis.

Hemostasis devices that are wrapped around a patient's limb at a site on the limb where bleeding is to be stopped, and which include one or more inflatable balloons or bladders that target pressure at a vascular access site, are known in the art. Multiple embodiments of one such hemostasis device and methods of using such devices are described in U.S. Patent No. 7,498,477. Additional embodiments of such hemostasis devices and methods of using same are described in U.S. Patent Application No. 16/288,303, filed February 28, 2019. It should be understood that the devices and methods taught herein could be used or adapted for use with any of the hemostasis devices taught in the references noted above in this paragraph.

As discussed in the '477 Patent noted above, such hemostasis devices generally include a rigid member (e.g., a curved plate that slips into a band) and at least one inflatable balloon that, when inflated, expands in a direction away from the rigid member and presses into a targeted location on a patient's limb or other body part, thereby promoting hemostasis. Many of these devices have a dual-balloon design including a connection port that connects the chambers of the two balloons in fluid-flow connection, such that inflating one balloon will cause the fluid (e.g., air) to flow through the connection port and fill the other balloon. These connection ports are typically made via radio frequency ("RF") welding or bonding between faces of the adjacent balloons. In some instances these connection ports can fail, thus causing the balloon assembly of the hemostasis device to fail to properly inflate. The connection port design also requires multiple manufacturing steps and costly and time-consuming manual placement of components during the construction process. Accordingly, there is a need for improved balloon assembly structures and methods of constructing same.

The present invention describes various embodiments of improved balloon assembly structures, each of which omit the connection port between the balloons. Several of these embodiments are formed of two or more layers of material (e.g., vinyl or PVC) connected together via a single welded perimeter and then folded to form a balloon assembly that includes both the large balloon and small balloon at the same time with an open air channel between the two sections. Said another way, the two or more balloon chambers and the air channel that connects between the balloon chambers collectively form a contiguous air chamber, with each component of the contiguous air chamber having been at least partially formed by a single welding step. In an alternative embodiment according to the present invention, a plurality of balloons are formed and a multi-output connector splits the inflation tubing into the appropriate number of output connection tubes, each of which is separately routed into one of the plurality of balloons. In either approach, significantly fewer manufacturing steps are needed, placement of the components of the balloon assembly is simpler and more automatable, and the relatively-weak connection port is eliminated.

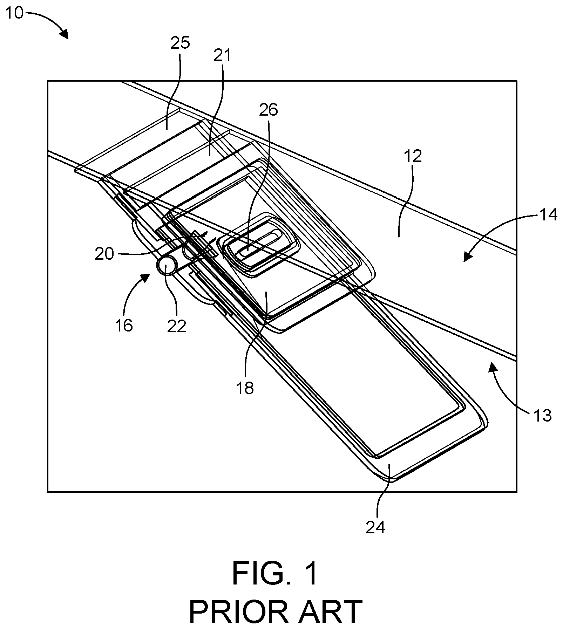

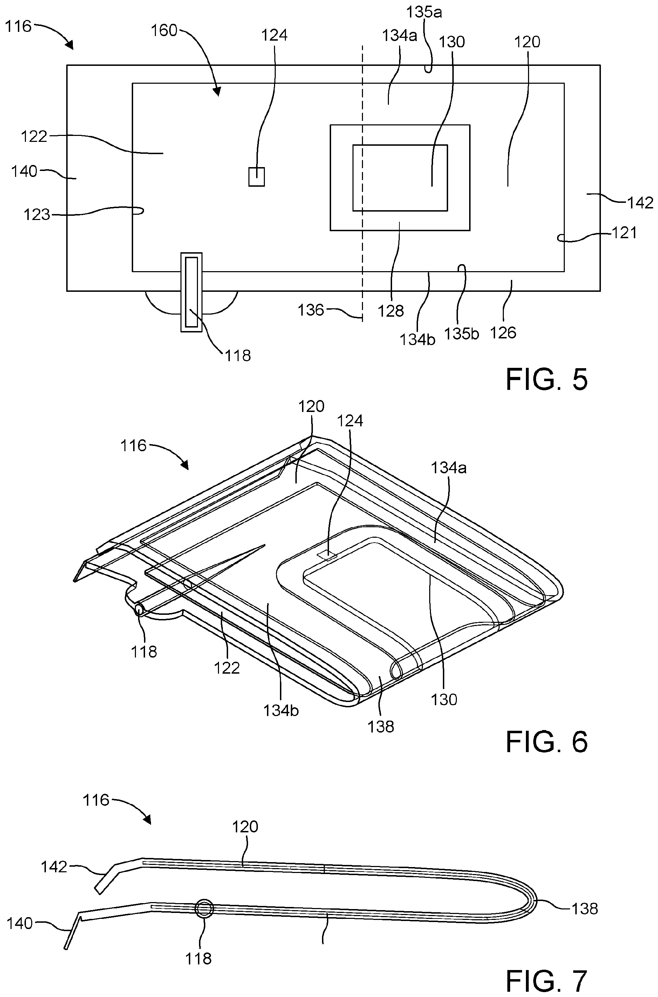

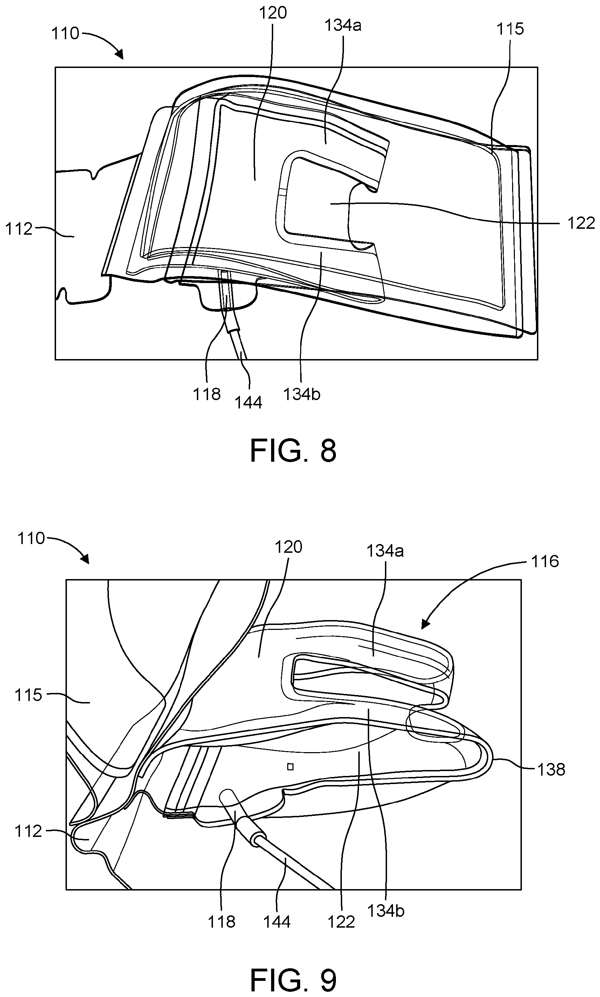

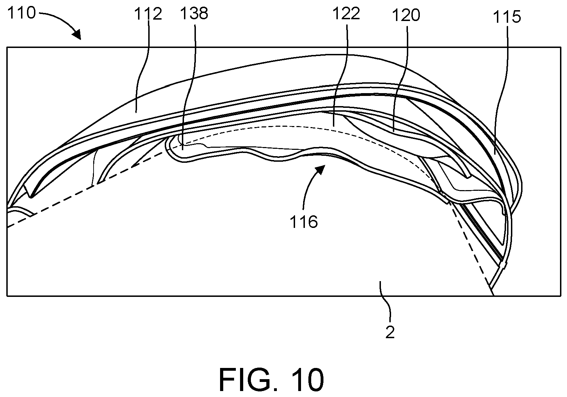

Referring now to FIGS. 1-23, various embodiments of balloon assemblies for hemostasis devices will be shown and described in detail. The hemostasis devices shown in the Figures are generally designed to be wrapped and secured in place around the arm 2 of a patient near the wrist to encourage hemostasis of the radial artery, as would be understood by a person having ordinary skill in the art. However, it should be understood that the concepts discussed in the present invention have applicability to other hemostasis devices that may be employed elsewhere on a patient's body, for example on any portion of any limb or the torso, neck, or head, and could be used for either arterial or venous hemostasis applications. Further, while it is generally desirable that the balloon assemblies according to the present invention be substantially transparent to permit visibility of the vascular access site (both for placement and for monitoring of complications), in alternative embodiments these balloon assemblies may be partially or entirely opaque.

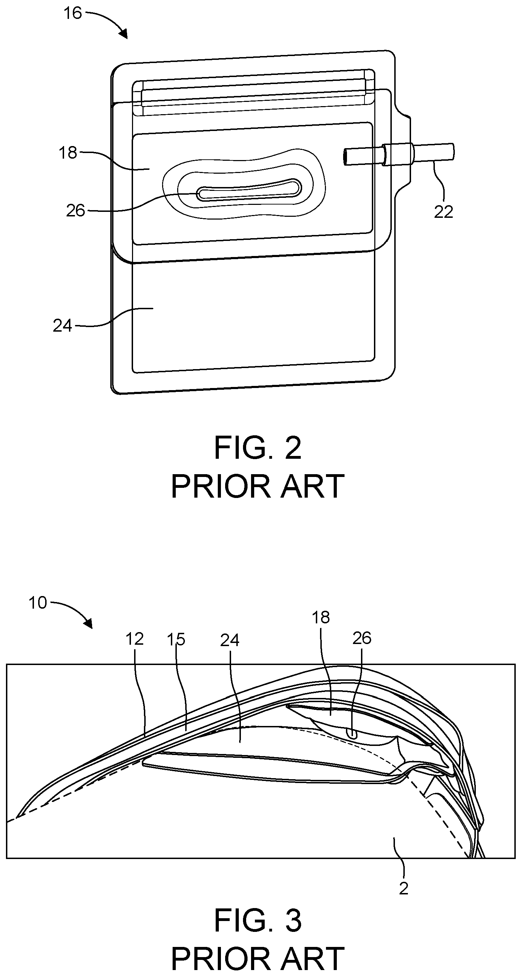

FIG. 1 is a partial perspective view of a hemostasis device 10 including a balloon assembly 16 according to the prior art, in an uninflated state. FIG. 2 shows this balloon assembly 16 by itself in an uninflated state. As shown in FIGS. 1 and 3, the balloon assembly 16 is attached to an interior side 13 of a band 12 that faces a patient's arm 2 when worn, the band 12 further comprising an an exterior side 14 opposing the interior side 13 and an insert plate 15 inserted within layers (not labeled) of the band 12. In this embodiment, the balloon assembly 16 comprises a small balloon 18 that is attached to the interior side 13 of the band 12 via an attachment hinge 21 and a large balloon 24 that is attached to the interior side 13 of the band 12 via an attachment hinge 25. A length of connection tubing 22 enters the balloon assembly 16 via an inlet 20, and a connection port 26 is formed between the small balloon 18 and large balloon 24 such that air entering the balloon assembly 16 via the connection tubing 22 can freely travel between the balloons 18,24 via the connection port 26. As noted above, the connection port 26 is typically RF welded and is subject to occasional failure when the balloon assembly 16 is inflated (as shown in FIG. 3).

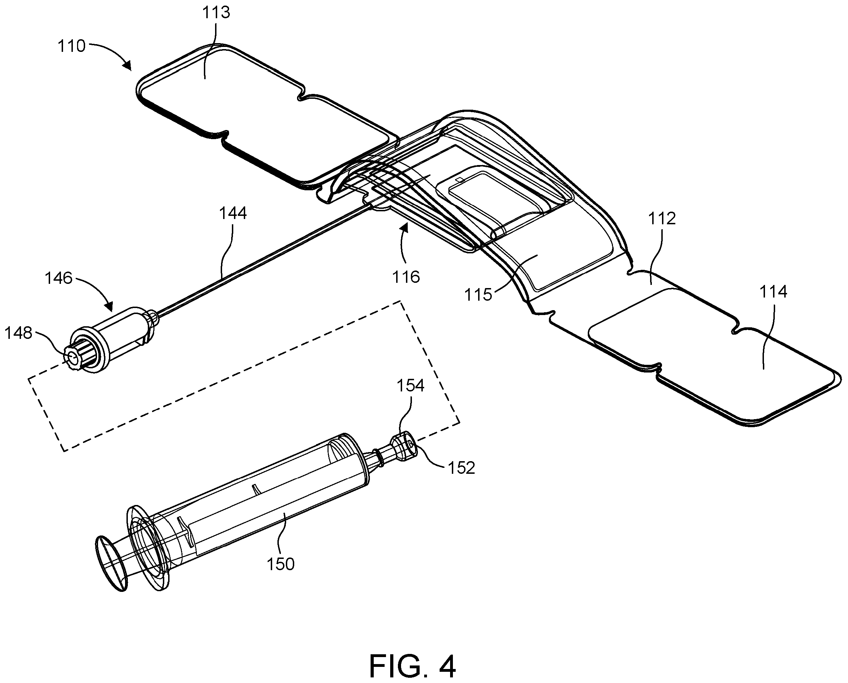

FIGS. 4-10 show various views of a balloon assembly 116 according to the present invention, and FIGS. 4 and 8-10 show the balloon assembly 116 attached to a hemostasis device 110 according to the present invention. FIGS. 4-10 show a "reverse end-fold" design for a balloon assembly 116 which has a single, welded outer perimeter 126 and a single, welded inner perimeter 128 around which a pair of air channels 134a,134b are formed. In this embodiment, both perimeters 126,128 are formed by laser welding the material layers together, but other construction methods are possible for connecting the material layers of the balloon assembly 116, for example but not limited to RF welding or gluing. In this embodiment, a cutout 130 is made within the inner perimeter 128 after it has been formed so that the channels 134a,134b are separate portions which are located on opposite sides of the cutout 130. In the alternative, the cutout 130 may be omitted so that the inner perimeter 128 surrounds a fully-welded region of two or more layers of material. In the present embodiment, the balloon assembly 116 is fully constructed by being folded about the fold line 136 so that a folded portion 138 is formed that includes the channels 134a, 134b, and a small balloon 120 is located atop a large balloon 122. In this and other embodiments according to the present invention, placing the balloon assembly 116 in its folded configuration aligns a first portion of the channel 134a atop a second portion of the channel 134a and aligns a first portion of the channel 134b atop a second portion of the channel 134b.

Via a single welding step of forming the two perimeters 126,128, the folded balloon assembly 116 of the present embodiment creates a dual-balloon structure comprising the small balloon 120, the large balloon 122, and the integrated air channels 134a,134b connecting the balloons 120,122, thereby achieving elimination of the weak welded connection port of the prior art devices while reducing the number of steps involved in the construction process. The small balloon 120, the large balloon 122, and the integrated air channels 134a,134b collectively comprise a contiguous air chamber 160, each component of which is formed at least in part by the single welding step. More particularly, the small balloon 120 has a perimeter 121, the large balloon 122 has a perimeter 123, and each of the air channels 134a,134b has a respective perimeter 135a,135b, and at least a portion of each of the perimeters 121,123,135a,135b-specifically, respective outer edge portions of each perimeter 121,123,135a,135b--is formed by the outer perimeter 126.

In the embodiment shown in FIGS. 4-10, the balloon assembly 116 is comprised of three layers of material around its outer perimeter 126 and two layers of material around its inner perimeter 128. In alternative embodiments, the balloon assembly 116 may be formed by attaching any plural number of material layers together about either or both of the outer perimeter 126 and inner perimeter 128, in different combinations, as would be appreciated by a person having ordinary skill in the art.

Turning back to the embodiment of FIGS. 4-10, the balloon assembly 116 is attached to the hemostasis device 110 via two separate attachment hinges 140,142, but in alternative embodiments a reverse end-fold balloon assembly design could have a single, shared attachment hinge by which the balloon assembly is attached to a hemostasis device. Further, while in the present embodiment two air channels 134a,134b are formed, this type of balloon assembly design could be formed with any number of air channels between the balloons 120,122.

In the present embodiment, the balloon assembly 116 includes an indicator 124 located on the large balloon 122 that is used to help the clinician properly align the hemostasis device 110 on the patient's body part (i.e., adjacent to or atop the vascular access site) before, during, or after inflation of the balloon assembly 116. Omitting a welded connection port from the balloon assembly 116 provides the additional benefit of enhancing the visibility of the indicator 124 and the underlying vascular access site, thereby increasing the likelihood that the clinician will perform the hemostasis procedure accurately. In alternative embodiments, the indicator 124 could be located elsewhere on the balloon assembly 116, located elsewhere on the hemostasis device 110 (e.g., on the band or rigid insert plate), or omitted entirely.

FIGS. 4 and 8-10 show the hemostasis device 110 comprising the balloon assembly 116 attached to a band 112 according to the invention of U.S. Patent Application No. 16/288,303. The hemostasis device 110 further comprises a rigid insert plate 115 that acts to direct the force of the inflated balloon assembly 116 towards the vascular access site, and complementary fastener patches 113,114 (e.g., of hook-and-loop type, though other fastener types are possible) located on the band 112 that are used to close and secure the band 112 around a patient's body part. In this embodiment, the balloon assembly 116 is inflatable via a connector and valve assembly 146 that is connected to an inlet 118 of the balloon assembly 116 for introduction of air into the balloon assembly 116 via a connection tubing 144. In this embodiment, the connector and valve assembly 146 is comprised of a hard connector having an integrated connector port 148 that mates only with the complementary inflator tip 152 of an inflator 150. In this embodiment, the inflator 150 comprises a collar 154 that surrounds the inflator tip 152 and prevents accidental or negligent misuse of the inflator 150, for example any attempt to insert the inflator tip 152 directly into an introducer sheath used during a vascular access procedure.

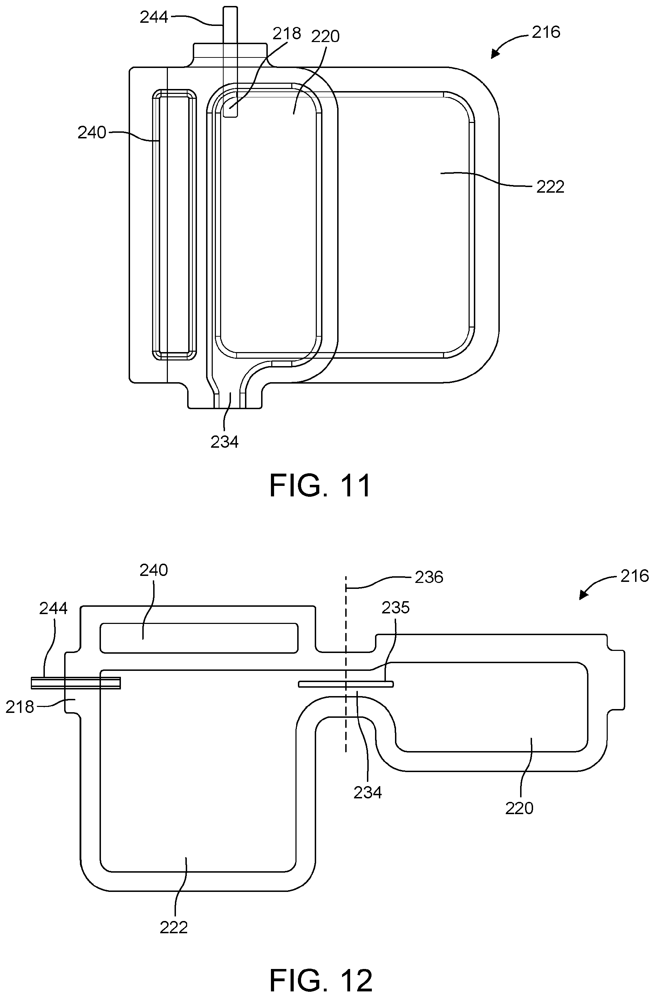

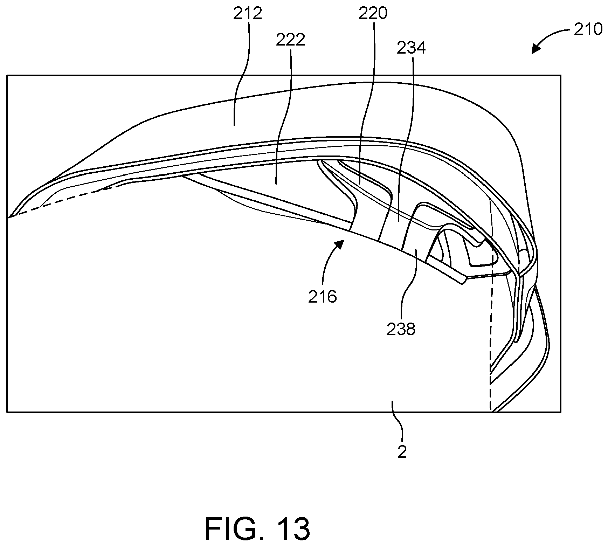

FIGS. 11-13 show a balloon assembly 216 according to another embodiment of the present invention, and FIG. 13 shows the balloon assembly 216 attached to a hemostasis device 210 having a band 212 that is attached around the arm 2 of a patient in the vicinity of the patient's wrist. FIGS. 11-13 show a "side fold" design of the balloon assembly 216 which has a single, outer laser-welded perimeter that forms a channel 234 that connects a small balloon 220 and a large balloon 222 together in fluid flow communication. A connection tubing 244 enters the balloon assembly 216 via an inlet 218 that allows for introduction of air into the balloon assembly 216. Via a single welding step, the folded balloon assembly 216 of the present embodiment creates a dual-balloon structure comprising the small balloon 220, the large balloon 222, and the integrated channel 234, thereby achieving elimination of the welded connection port of the prior art devices. As shown in FIG. 12, the balloon assembly 216 is completed by folding it about the fold line 236 to create a folded portion 238, and such that a first portion of the channel 234 is placed atop a second portion of the channel. In this embodiment, the balloon assembly 216 is attached to the hemostasis device 210 via a single attachment hinge 240, but in alternative embodiments a side fold balloon assembly design could have two separate attachment hinges by which the balloon assembly is attached to a hemostasis device. As shown in FIG. 12 and further described in detail below, a "breather" strip 235, layer, or other component can optionally be included within the channel 234 to help prevent material adhesion between the two layers of the balloon assembly 216 or kinking of the channel 234, thus reducing the likelihood that the channel 234 will fail to inflate, causing the balloon assembly 216 to fail.

In some embodiments according to the present invention, when the balloon assembly is attached to the band of the hemostasis device in its intended configuration, there is some possibility that the folded channel could become tightly creased such that airflow is all or partially kinked off between the balloons. In the various embodiments described herein, one or more pieces of secondary material can optionally be included within each channel to help hold the channel open. These "breather strips" may be one or more additional pieces of material included within the channel, which may be comprised of either air-permeable or air-impermeable materials. Alternatively, or in addition, the channel(s) can be partially held open along their edge(s) by creating height along the one or more perimeter(s) of the balloon assembly construction using: one or more additional layer(s) of material; a glue line; and/or an extruded bead or weld line resulting from a RF welding process, along the one or more perimeter(s).

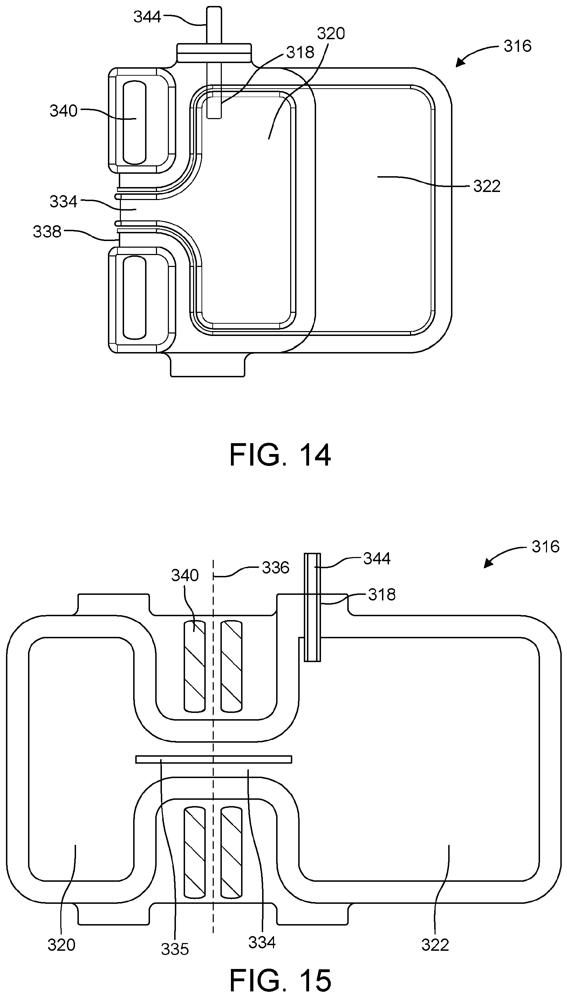

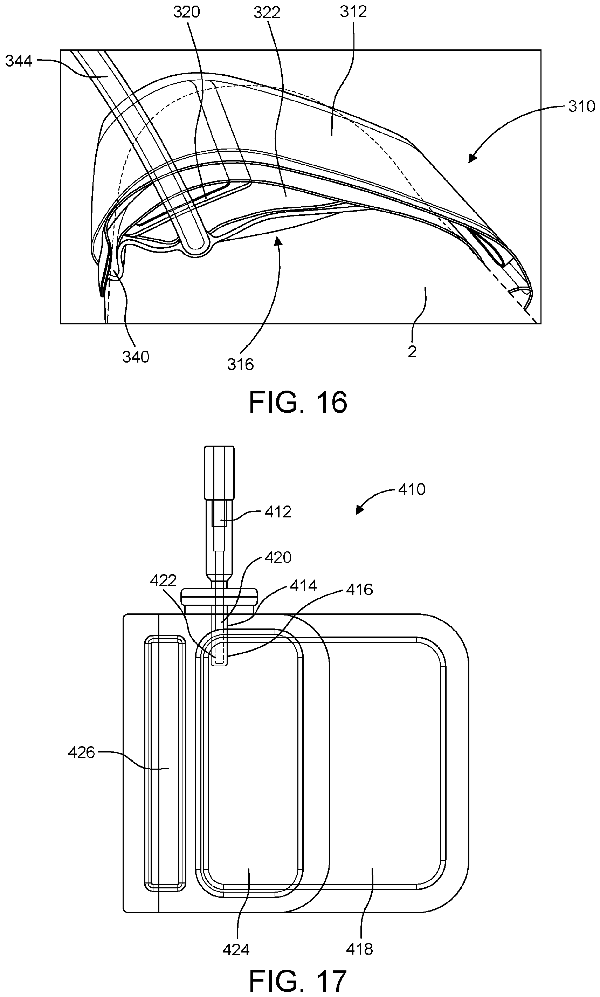

FIGS. 14-16 show a balloon assembly 316 according to another embodiment of the present invention, and FIG. 16 shows the balloon assembly 316 attached to a hemostasis device 310 having a band 312 that is attached around the arm 2 of a patient in the vicinity of the patient's wrist. FIGS. 14 and 15 show a "center vent" folded design that is a form of end fold design in which an air channel 334 is created in the center of a side edge of the balloon assembly 316. In this embodiment, the balloon assembly 316 has single, outer laser-welded perimeter that forms the single air channel 334 connecting a small balloon 320 and large balloon 322 together in fluid flow communication. In this embodiment, the remainder of the side edge that includes the air channel 334 is sealed and serves as an attachment hinge 340 for connecting the balloon assembly 316 to the hemostasis device 310. A connection tubing 344 enters the balloon assembly 316 via an inlet 318 that allows for introduction of air into the balloon assembly 316. Via a single welding step, the folded balloon assembly 316 of the present embodiment creates a dual-balloon structure comprising the small balloon 320, the large balloon 322, and the integrated channel 334, thereby achieving elimination of the welded connection port of the prior art devices. As shown in FIG. 15, the balloon assembly 316 is completed by folding it about the fold line 336 to create a folded portion 338, and such that a first portion of the channel 334 is placed atop a second portion of the channel 334. In this embodiment, the balloon assembly 316 is attached to the hemostasis device 310 via a single attachment hinge 340 that is located along the same edge of the balloon assembly 316 as the channel 334. In alternative embodiments, a "center-vent" end-fold balloon assembly design could have one or two separate attachment hinges located on the edge of the balloon assembly 316 opposing the edge where the channel 334 is located (i.e., on the edges of the small balloon 320 and/or large balloon 322 shown on the right side in the view of FIG. 14). In this embodiment, a breather strip 335 may be optionally included within the channel 334.

FIG. 17 shows another embodiment of a balloon assembly 410 according to the present invention. In this embodiment, rather than a single folded balloon design with a direct, integral air channel, the balloon assembly 410 is formed of two separate balloons, a small balloon 424 and a large balloon 418, that are not directly connected together in fluid flow communication via an integral channel. Instead, a Y-shaped connector 412 that has a single air inlet and two air outlets is used, with a first connection tubing 414 routed between one of the air outlets (not labeled) of the Y-shaped connector 412 and an inlet 416 on the large balloon 418 and a second connection tubing 420 routed between the other of the air outlets (not labeled) of the Y-shaped connector 412 and an inlet 422 of the small balloon 424. In this embodiment, the balloon assembly 410 is attachable to a hemostasis device via a single attachment hinge 426, but in alternative embodiments a multi-balloon assembly design with separated air input lines could have separate attachment hinges by which the balloon assembly is attached to a hemostasis device.

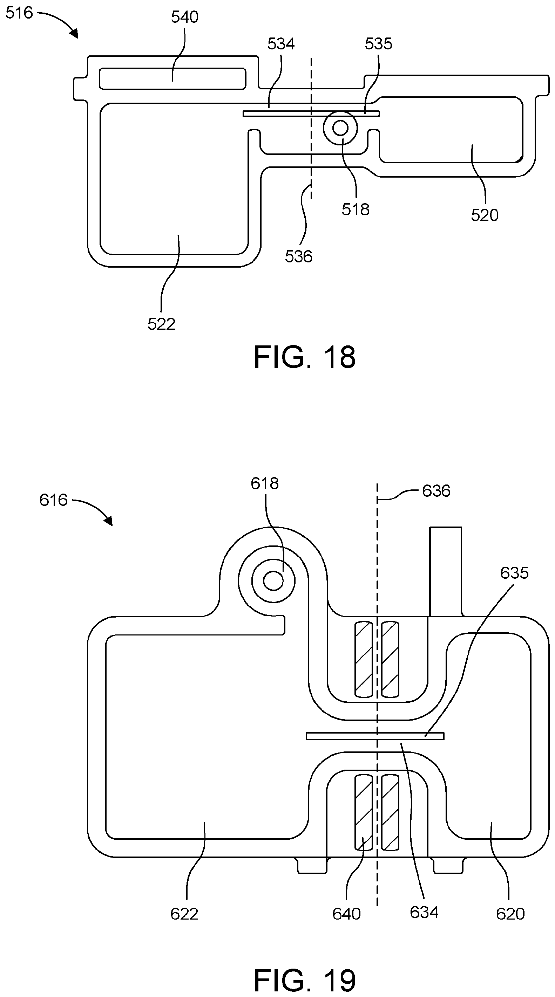

Rather than using connection tubing to feed air through an inlet that is located along a side edge of the balloon assembly, balloon assemblies according to the present invention could also utilize a "chimney"-style port that is routed perpendicularly to the surfaces of the balloon(s). FIGS. 18 and 19 show, respectively, schematic views of a side-fold balloon assembly 516 and a "center-vent" end-fold balloon assembly 616, which include respective chimney ports 518,618 that form the air inlet for the respective balloon assembly 516,616. It should be understood that balloon assembly 516 is otherwise functionally similar to balloon assembly 216 of FIGS. 11-13 and that balloon assembly 616 is otherwise functionally similar to balloon assembly 316 of FIGS. 14-16, with like parts in the embodiments of FIGS. 18 and 19 labeled with reference numerals increased by a value of 300 with respect to the respective related embodiment.

While the embodiments discussed above are designed as two-balloon structures, additional folds or split air lines could be used to form a balloon assembly having any number of balloons or separate air chambers in accordance with the inventive concepts taught herein. Further, in accordance with any of the embodiments, structures, concepts, or methods taught herein, the channel(s) or air passages between the balloons could be of any number, could be of any non-linear shape (e.g., angled, zig-zagged, curved), and/or could split, combine, or both. In alternative embodiments, any connection tubing could be replaced by a "chimney port" or hose barb.

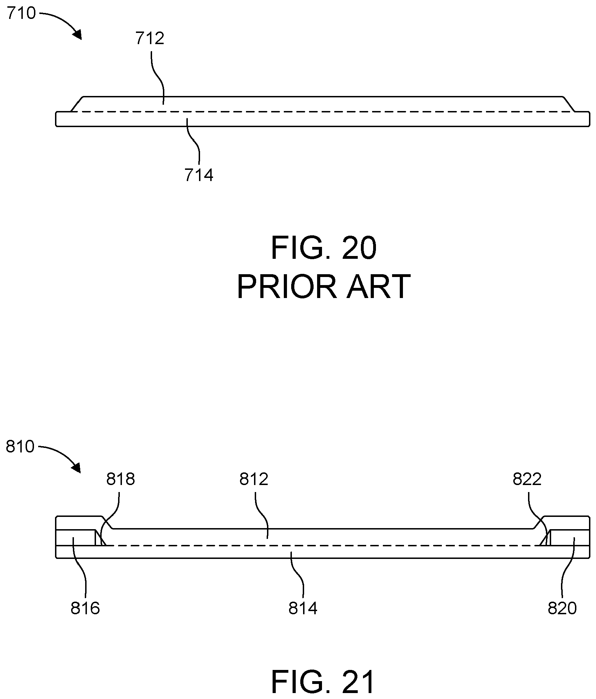

Another drawback with the structure of existing balloon assemblies is expansion defects or failures caused by the top and bottom layers of balloons adhering to another and failing to properly separate and permit the balloon to inflate after long periods of having been adjacent to another (i.e., after long periods of the balloon being uninflated). Referring now to FIG. 20, a sectional view of a balloon assembly 710 according to the prior art is shown in an uninflated state, with a top layer 712 and a bottom layer 714 thereof shown adjacent to another with no air gap or space between the layers 712,714.

In some embodiments according to the present invention, this expansion failure is addressed by including spacer(s), strip(s), and/or additional layer(s) of material between the top and bottom layers of the balloon, or otherwise forming space(s) between the layers of material. Materials can be added within formed air channel(s) to prevent these air paths from sealing off when the balloon assembly is folded. These "breather strips" are formed from air-permeable materials, including but not limited to felt, thread, paper, and porous plastic. In alternative embodiments, non-permeable materials can be placed such that they prop open air channel(s), thus allowing air to pass through the channel(s) adjacent to the material. Suitable non-permeable materials include but are not limited to tubing, stickers (adhesive backed paper), flexible sheets of either similar or dissimilar material to the material of the flexible sheet of the balloon, and/or cured glue. Holding channel(s) open at their edges via non-permeable materials, as shown in the example of FIG. 21 below, achieves the same effect as inserting air-permeable "breather strips" between layers of the balloon to form air channel(s). These space(s) may be located in the vicinity of the air injection port such that when air is injected into the balloon(s) the space serves as a trigger that helps peel apart any adhesions between the layers as air continues to flow into the balloon(s). Breather strips may be of any suitable cross-sectional shape, including but not limited to circular, oval, or rectangular.

FIG. 21 shows a sectional view of a balloon assembly 810 according to the present invention in an uninflated state, with a top layer 812 and a bottom layer 814 thereof shown mostly adjacent to another, but further including spacers 816,820 along the side edges (perimeter) of the balloon assembly 810 that introduce respective air gaps 818,822 adjacent to the spacers 816,820, such that when air is introduced into the balloon assembly 810 between the top layer 812 and bottom layer 814, the air gaps 818,822 created by the spacers 816,820 serve as air flow paths that promote proper inflation of the balloon assembly 810, overcoming any adherence between the two layers 812,814. It should be understood that the assembly shown in FIG. 21 could be representative of the entire cross-section of a balloon assembly, or instead of one channel of a multi-channel balloon assembly. It should further be understood that a spacer or third layer of material could be included around one edge (e.g., outer perimeter) of the assembly or channel, while another edge (e.g., inner perimeter) is comprised of only two material layers.

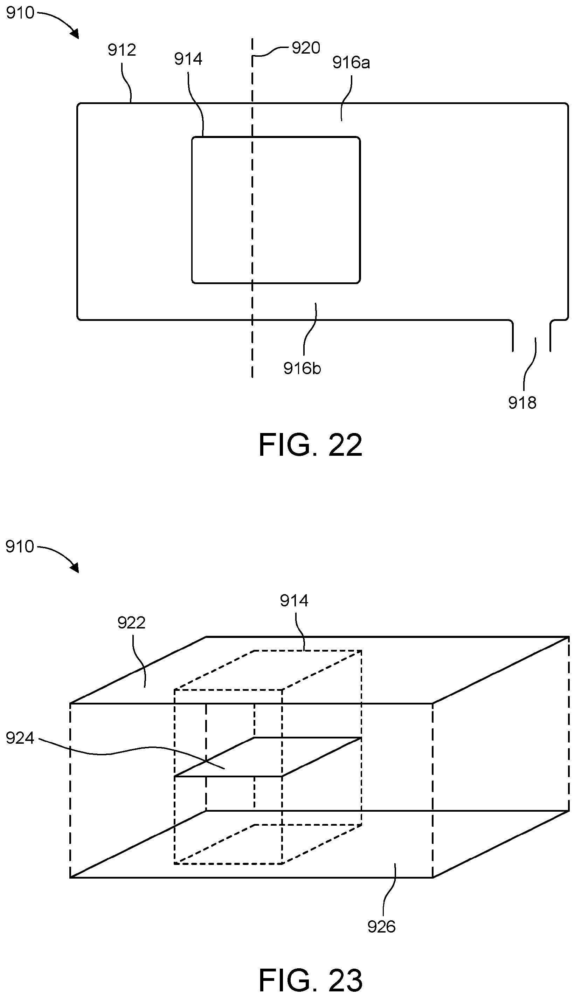

FIGS. 22 and 23 schematically depict a balloon assembly 910 that includes one (or more) intermediate layer(s) used as spacer(s) between top and bottom layers thereof. FIG. 22 schematically depicts an unfolded balloon assembly 910 formed by bonding or laser welding a top layer 922, middle layer 924, and bottom layer 926 together via a weld line 912 located around an exterior perimeter of the balloon assembly 910, and a weld line 914 located around an interior perimeter of the balloon assembly 910, with the top layer 922 and bottom layer 926 welded (or bonded) together via the exterior weld line 912 at the perimeter (leaving space 918 for an air inlet), and the middle layer 924 welded (or bonded) to both of the top layer 922 and the bottom layer 926 via weld line 914 to act as a spacer therebetween (the squares projecting upwardly and downwardly from the middle layer 924 in FIG. 23 depict the weld/bonding locations to the respective layers 922,926). The weld line 914 creates a pair of channels 916a,916b on either side thereof, such that when the balloon assembly 910 is folded about fold line 920 to form a completed balloon, air can travel through these channels 916a,916b between the two formed balloon chambers. The balloon assembly 910 of the present embodiment has both two- and three-layer portions, with the three-layer portions acting to prevent expansion failure in the remainder of the balloon assembly 910. In this embodiment, once folded it is possible to cut out the interior area of the center welded area (the area inside weld line 914), since this is not necessary for proper functioning of the balloon assembly 910. By this method, an "end-fold" balloon assembly-similar to the balloon assembly 116 of FIGS. 4-10-is constructed.

While the principles of the claimed invention have been described above in connection with specific embodiment(s), it is to be clearly understood that this description is made only by way of example and not as a limitation of the scope of the invention, as set forth in the appended claims.

## Claims

1. A balloon assembly (16, 116, 216, 316, 410, 516, 616, 710, 810, 910) for a hemostasis device (10, 110, 210, 310), the balloon assembly (16, 116, 216, 316, 410, 516, 616, 710, 810, 910) comprising:
a first chamber (120);
a second chamber (122); and
at least one channel (134a-b, 234, 334, 916a-b) that is in fluid flow communication between the first chamber and the second chamber, wherein a single perimeter of attachment between a first layer of material and a second layer of material defines at least a portion of a perimeter of the first chamber, at least a portion of a perimeter of the second chamber, and at least a portion of a perimeter of the at least one channel (134a-b, 234, 334, 916a-b), **characterized in that** at least a portion of the first chamber overlays at least a portion of the second chamber, and **in that** the at least one channel is folded.

2. The balloon assembly (16, 116, 216, 316, 410, 516, 616, 710, 810, 910) of claim 1, wherein the single perimeter of attachment defines the entireties of the perimeters of the first chamber and the second chamber.

3. The balloon assembly (16, 116, 216, 316, 410, 516, 616, 710, 810, 910) of claim 2, wherein the single perimeter of attachment defines the entirety of the perimeter of the at least one channel (134a-b, 234, 334, 916a-b).

4. The balloon assembly (16, 116, 216, 316, 410, 516, 616, 710, 810, 910) of any of claims 1-3, the balloon assembly (16, 116, 216, 316, 410, 516, 616, 710, 810, 910) further comprising an exterior edge, wherein the at least one channel (134a-b, 234, 334, 916a-b) is folded around the exterior edge.

5. The balloon assembly (16, 116, 216, 316, 410, 516, 616, 710, 810, 910) of claim 4, wherein a first portion of the at least one channel (134a-b, 234, 334, 916a-b) overlays a second portion of the at least one channel (134a-b, 234, 334, 916a-b).

6. The balloon assembly (16, 116, 216, 316, 410, 516, 616, 710, 810, 910) of claim 1, further comprising at least one piece of secondary material located within the at least one channel (134a-b, 234, 334, 916a-b).

7. The balloon assembly (16, 116, 216, 316, 410, 516, 616, 710, 810, 910) of claim 6, wherein the at least one piece of secondary material is formed of a gas-permeable material.

8. The balloon assembly (16, 116, 216, 316, 410, 516, 616, 710, 810, 910) of claim 6, wherein the at least one piece of secondary material is formed of a gas-impermeable material.

9. The balloon assembly (16, 116, 216, 316, 410, 516, 616, 710, 810, 910) of claim 6, wherein the at least one piece of secondary material has a circular cross-sectional shape.

10. The balloon assembly (16, 116, 216, 316, 410, 516, 616, 710, 810, 910) of claim 1, further comprising a third layer of material that is at least partially located between and attached to the first layer of material and the second layer of material.

11. The balloon assembly (16, 116, 216, 316, 410, 516, 616, 710, 810, 910) of claim 1, the single perimeter of attachment comprising an outer perimeter (126), the balloon assembly (16, 116, 216, 316, 410, 516, 616, 710, 810, 910) further comprising an inner perimeter (128) of attachment between the first layer and the second layer, the inner perimeter (128) being located interior to the outer perimeter (126).

12. The balloon assembly (16, 116, 216, 316, 410, 516, 616, 710, 810, 910) of claim 11, wherein the outer perimeter (126) and the inner perimeter (128) form a first air channel (134a-b, 234, 334, 916a-b) and a second air channel (134a-b, 234, 334, 916a-b) between the first chamber and the second chamber.

13. The balloon assembly (16, 116, 216, 316, 410, 516, 616, 710, 810, 910) of claim 12, further comprising a cutout region interior to the inner perimeter (128) from which portions of the first layer of material and second layer of material are absent.

14. The balloon assembly (16, 116, 216, 316, 410, 516, 616, 710, 810, 910) of claim 1, further comprising an inlet (20,118, 218, 318, 416, 422) located along the perimeter through which a fluid can be introduced into an interior of the balloon assembly (16, 116, 216, 316, 410, 516, 616, 710, 810, 910).

15. The balloon assembly (16, 116, 216, 316, 410, 516, 616, 710, 810, 910) of claim 14, wherein the inlet (20,118, 218, 318, 416, 422) is in the form of a hollow cylindrical tubing.

## Patentansprüche

1. Ballonanordnung (16, 116, 216, 316, 410, 516, 616, 710, 810, 910) für eine hämostatische Vorrichtung (10, 110, 210, 310), wobei die Ballonanordnung (16, 116, 216, 316, 410, 516, 616, 710, 810, 910) umfasst:
eine erste Kammer (120);
eine zweite Kammer (122); und
mindestens einen Kanal (134a-b, 234, 334, 916a-b), der in Fluidströmungsverbindung zwischen der ersten Kammer und der zweiten Kammer steht, wobei ein einzelner Umfang der Anbringung zwischen einer ersten Materialschicht und einer zweiten Materialschicht mindestens einen Abschnitt von einem Umfang der ersten Kammer, mindestens einen Abschnitt von einem Umfang der zweiten Kammer und mindestens einen Abschnitt von einem Umfang des mindestens einen Kanals (134a-b, 234, 334, 916a-b) definiert,
**dadurch gekennzeichnet, dass** mindestens ein Abschnitt von der ersten Kammer mindestens einen Abschnitt der zweiten Kammer überlagert, und dadurch, dass der mindestens eine Kanal gefaltet ist.

2. Ballonanordnung (16, 116, 216, 316, 410, 516, 616, 710, 810, 910) nach Anspruch 1, wobei der einzelne Umfang der Anbringung die Gesamtheit der Umfänge von der ersten Kammer und der zweiten Kammer definiert.

3. Ballonanordnung (16, 116, 216, 316, 410, 516, 616, 710, 810, 910) nach Anspruch 2, wobei der einzelne Umfang der Anbringung die Gesamtheit von dem Umfang des mindestens einen Kanals (134a-b, 234, 334, 916a-b) definiert.

4. Ballonanordnung (16, 116, 216, 316, 410, 516, 616, 710, 810, 910) nach einem der Ansprüche 1 bis 3, wobei die Ballonanordnung (16, 116, 216, 316, 410, 516, 616, 710, 810, 910) ferner eine äußere Kante umfasst, wobei der mindestens eine Kanal (134a-b, 234, 334, 916a-b) um die äußere Kante herum gefaltet ist.

5. Ballonanordnung (16, 116, 216, 316, 410, 516, 616, 710, 810, 910) nach Anspruch 4, wobei ein erster Abschnitt des mindestens einen Kanals (134a-b, 234, 334, 916a-b) einen zweiten Abschnitt des mindestens einen Kanals (134a-b, 234, 334, 916a-b) überlagert.

6. Ballonanordnung (16, 116, 216, 316, 410, 516, 616, 710, 810, 910) nach Anspruch 1, die ferner mindestens ein Stück Sekundärmaterial umfasst, welches innerhalb des mindestens einen Kanals (134a-b, 234, 334, 916a-b) angeordnet ist.

7. Ballonanordnung (16, 116, 216, 316, 410, 516, 616, 710, 810, 910) nach Anspruch 6, wobei das mindestens eine Stück Sekundärmaterial aus einem gasdurchlässigen Material gebildet ist.

8. Ballonanordnung (16, 116, 216, 316, 410, 516, 616, 710, 810, 910) nach Anspruch 6, wobei das mindestens eine Stück Sekundärmaterial aus einem gasundurchlässigen Material gebildet ist.

9. Ballonanordnung (16, 116, 216, 316, 410, 516, 616, 710, 810, 910) nach Anspruch 6, wobei das mindestens eine Stück Sekundärmaterial eine kreisförmige Querschnittsform aufweist.

10. Ballonanordnung (16, 116, 216, 316, 410, 516, 616, 710, 810, 910) nach Anspruch 1, die ferner eine dritte Materialschicht umfasst, welche mindestens teilweise zwischen der ersten Materialschicht und der zweiten Materialschicht angeordnet und daran angebracht ist.

11. Ballonanordnung (16, 116, 216, 316, 410, 516, 616, 710, 810, 910) nach Anspruch 1, wobei der einzelne Umfang der Anbringung einen äußeren Umfang (126) umfasst, wobei die Ballonanordnung (16, 116, 216, 316, 410, 516, 616, 710, 810, 910) ferner einen inneren Umfang (128) der Anbringung zwischen der ersten Schicht und der zweiten Schicht umfasst, wobei der innere Umfang (128) innerhalb des äußeren Umfangs (126) angeordnet ist.

12. Ballonanordnung (16, 116, 216, 316, 410, 516, 616, 710, 810, 910) nach Anspruch 11, wobei der äußere Umfang (126) und der innere Umfang (128) einen ersten Luftkanal (134a-b, 234, 334, 916a-b) und einen zweiten Luftkanal (134a-b, 234, 334, 916a-b) zwischen der ersten Kammer und der zweiten Kammer bilden.

13. Ballonanordnung (16, 116, 216, 316, 410, 516, 616, 710, 810, 910) nach Anspruch 12, die ferner einen ausgeschnittenen Bereich innerhalb des inneren Umfangs (128) umfasst, von dem Abschnitte von der ersten Materialschicht und der zweiten Materialschicht abwesend sind.

14. Ballonanordnung (16, 116, 216, 316, 410, 516, 616, 710, 810, 910) nach Anspruch 1, die ferner einen Einlass (20, 118, 218, 318, 416, 422) umfasst, welcher entlang des Umfangs angeordnet ist, durch den ein Fluid in das Innere der Ballonanordnung (16, 116, 216, 316, 410, 516, 616, 710, 810, 910) eingeführt werden kann.

15. Ballonanordnung (16, 116, 216, 316, 410, 516, 616, 710, 810, 910) nach Anspruch 14, wobei der Einlass (20, 118, 218, 318, 416, 422) die Form eines hohlen zylindrischen Rohres aufweist.

## Revendications

1. Ensemble de ballonnets (16, 116, 216, 316, 410, 516, 616, 710, 810, 910) pour un dispositif d'hémostase (10, 110, 210, 310), l'ensemble de ballonnets (16, 116, 216, 316, 410, 516, 616, 710, 810, 910) comprenant :
une première chambre (120) ;
une deuxième chambre (122), et
au moins un canal (134a-b, 234, 334, 916a-b) qui est en communication d'écoulement de fluide entre la première chambre et la deuxième chambre, dans lequel un unique périmètre de fixation entre une première couche de matériau et une deuxième couche de matériau définit au moins une partie d'un périmètre de la première chambre, au moins une partie d'un périmètre de la deuxième chambre, et au moins une partie d'un périmètre de l'au moins un canal (134a-b, 234, 334, 916a-b), **caractérisé en ce qu'**au moins une partie de la première chambre recouvre au moins une partie de la deuxième chambre, et **en ce que** l'au moins un canal est plié.

2. Ensemble de ballonnets (16, 116, 216, 316, 410, 516, 616, 710, 810, 910) selon la revendication 1, dans lequel l'unique périmètre de fixation définit les totalités des périmètres de la première chambre et de la deuxième chambre.

3. Ensemble de ballonnets (16, 116, 216, 316, 410, 516, 616, 710, 810, 910) selon la revendication 2, dans lequel l'unique périmètre de fixation définit la totalité du périmètre de l'au moins un canal (134a-b, 234, 334, 916a-b).

4. Ensemble de ballonnets (16, 116, 216, 316, 410, 516, 616, 710, 810, 910) selon l'une quelconque des revendications 1 à 3, l'ensemble de ballonnets (16, 116, 216, 316, 410, 516, 616, 710, 810, 910) comprenant en outre un bord extérieur, dans lequel l'au moins un canal (134a-b, 234, 334, 916a-b) est plié autour du bord extérieur.

5. Ensemble de ballonnets (16, 116, 216, 316, 410, 516, 616, 710, 810, 910) selon la revendication 4, dans lequel une première partie de l'au moins un canal (134a-b, 234, 334, 916a-b) recouvre une deuxième partie de l'au moins un canal (134a-b, 234, 334, 916a-b).

6. Ensemble de ballonnets (16, 116, 216, 316, 410, 516, 616, 710, 810, 910) selon la revendication 1, comprenant en outre au moins une pièce de matériau secondaire située au sein de l'au moins un canal (134a-b, 234, 334, 916a-b).

7. Ensemble de ballonnets (16, 116, 216, 316, 410, 516, 616, 710, 810, 910) selon la revendication 6, dans lequel l'au moins une pièce de matériau secondaire est formée d'un matériau perméable aux gaz.

8. Ensemble de ballonnets (16, 116, 216, 316, 410, 516, 616, 710, 810, 910) selon la revendication 6, dans lequel l'au moins une pièce de matériau secondaire est formée d'un matériau imperméable aux gaz.

9. Ensemble de ballonnets (16, 116, 216, 316, 410, 516, 616, 710, 810, 910) selon la revendication 6, dans lequel l'au moins une pièce de matériau secondaire possède une forme de section transversale circulaire.

10. Ensemble de ballonnets (16, 116, 216, 316, 410, 516, 616, 710, 810, 910) selon la revendication 1, comprenant en outre une troisième couche de matériau qui est au moins partiellement située entre la première couche de matériau et la deuxième couche de matériau et fixée à celles-ci.

11. Ensemble de ballonnets (16, 116, 216, 316, 410, 516, 616, 710, 810, 910) selon la revendication 1, l'unique périmètre de fixation comprenant un périmètre externe (126), l'ensemble de ballonnets (16, 116, 216, 316, 410, 516, 616, 710, 810, 910) comprenant en outre un périmètre interne (128) de fixation entre la première couche et la deuxième couche, le périmètre interne (128) étant situé à l'intérieur du périmètre externe (126).

12. Ensemble de ballonnets (16, 116, 216, 316, 410, 516, 616, 710, 810, 910) selon la revendication 11, dans lequel le périmètre externe (126) et le périmètre interne (128) forment un premier canal d'air (134a-b, 234, 334, 916a-b) et un deuxième canal d'air (134a-b, 234, 334, 916a-b) entre la première chambre et la deuxième chambre.

13. Ensemble de ballonnets (16, 116, 216, 316, 410, 516, 616, 710, 810, 910) selon la revendication 12, comprenant en outre une région de découpe intérieure au périmètre interne (128) à partir de laquelle des parties de la première couche de matériau et de la deuxième couche de matériau sont absentes.

14. Ensemble de ballonnets (16, 116, 216, 316, 410, 516, 616, 710, 810, 910) selon la revendication 1, comprenant en outre une entrée (20, 118, 218, 318, 416, 422) située le long du périmètre, par laquelle un fluide peut être introduit dans un intérieur de l'ensemble de ballonnets (16, 116, 216, 316, 410, 516, 616, 710, 810, 910).

15. Ensemble de ballonnets (16, 116, 216, 316, 410, 516, 616, 710, 810, 910) selon la revendication 14, dans lequel l'entrée (20, 118, 218, 318, 416, 422) se présente sous la forme d'un tube cylindrique creux.
